(19) Europäisches Patentamt

European Patent Office

Office européen des brevets

(11) **EP 1 358 839 B1**

(12) **EUROPÄISCHE PATENTSCHRIFT**

(45) Veröffentlichungstag und Bekanntmachung des Hinweises auf die Patenterteilung:
**23.02.2005 Patentblatt 2005/08**

(51) Int Cl.$^7$: **A61B 3/10**, A61B 3/107

(21) Anmeldenummer: **02405272.2**

(22) Anmeldetag: **05.04.2002**

(54) **Vorrichtung und Verfahren zur Bestimmung von geometrischen Messwerten eines Auges**

Apparatus and method for determining geometric measured values for an eye

Dispositif et procédé de determination des valeurs de mesure géométriques d'un oeil

(84) Benannte Vertragsstaaten:
**CH DE ES FR GB IT LI**

(43) Veröffentlichungstag der Anmeldung:
**05.11.2003 Patentblatt 2003/45**

(73) Patentinhaber: **SIS AG Surgical Instrument Systems**
**2555 Brügg (CH)**

(72) Erfinder: **Rathjen, Christian**
**28197 Bremen (DE)**

(74) Vertreter: **Vogel, Dany et al**
**Isler & Pedrazzini AG**
**Gotthardstrasse 53**
**Postfach 6940**
**8023 Zürich (CH)**

(56) Entgegenhaltungen:
WO-A-01/62140          US-A- 5 867 250
US-A- 5 886 767        US-B1- 6 234 631

EP 1 358 839 B1

**Beschreibung**

Technisches Gebiet

Die vorliegende Erfindung betrifft eine Vorrichtung und ein Verfahren zur Bestimmung von geometrischen Messwerten eines Auges. Die Erfindung betrifft insbesondere eine Vorrichtung und ein Verfahren zur Bestimmung von geometrischen Messwerten eines menschlichen Auges, in welchen mittels eines Lichtprojektors ein Strahlenbündel durch einen Querschnittsteil des Auges projiziert wird, in welchen mittels Bilderfassungsmitteln ein erstes Abbild von mindestens einem Teilgebiet des durch den Lichtprojektor beleuchteten Querschnittsteils unter einem ersten Beobachtungswinkel, aus einer ersten Position ausserhalb des Strahlenbündels erfasst wird, und in welchen mittels der Bilderfassungsmittel ein zweites Abbild des Teilgebiets unter einem zweiten Beobachtungswinkel, aus einer zweiten Position ausserhalb des Strahlenbündels erfasst wird.

Stand der Technik

In der Patentschrift US 6,234,631 wird ein Verfahren zur Vermessung der hinteren und vorderen Hornhautoberfläche und der Hornhautdicke des Auges beschrieben. Im Verfahren gemäss US 6,234,631 werden eine frontale Kamera zur Aufnahme einer Frontalansicht und symmetrisch dazu eine linke und eine rechte Kamera zur Aufnahme von zwei Seitenansichten des Auges verwendet. Die linke und die rechte Kamera sind jeweils in einem 45-Grad-Winkel zur optischen Achse der frontalen Kamera orientiert. Im Verfahren gemäss US 6,234,631 wird ein Lichtmuster in der Form eines Kreuzes, ähnlich wie zwei senkrecht zueinander stehende Lichtspalten, auf die Hornhaut projiziert und die Iris des Auges wird zur Kontrastierung der Pupille mit Infrarotlicht beleuchtet. Mit der frontalen Kamera wird der horizontale Teil des Lichtkreuzes und mit der linken und der rechten Kamera wird jeweils der vertikale Teil des Lichtkreuzes aufgenommen. Gleichzeitig wird durch jede der drei Kameras ein Bild der Pupille aufgenommen. Aus den Pupillenbildern wird der Pupillenumriss aus der Sicht jeder der drei Kameras bestimmt. Aus den Aufnahmen des Lichtkreuzes und unter Annahme einer ungefähren Hornhautoberfläche wird gemäss US 6,234,631 auf der Grundlage des Strahlengangs Lichtquelle-Hornhaut-Kamera, mittels so genanntem Ray Tracing, ein erster Näherungswert für die Hornhautdicke berechnet. Dieser erste Näherungswert dient als Ausgangswert für ein iteratives Verfahren, in welchem ausgehend von der vorgängig bestimmten Topographie der vorderen Hornhautoberfläche die Hornhautdicke und die Topographie der hinteren Hornhautoberfläche bestimmt wird. Die Bestimmung der Topographie der vorderen Hornhautoberfläche erfolgt durch iterative Berechnung aus Aufnahmen von Spiegelungen einer Placidoscheibe auf der Hornhaut, die mittels der drei Kameras aufgenommen werden. Die Hornhautdicke und die Topographie der hinteren Hornhautoberfläche werden gemäss US 6,234,631 iterativ auf der Grundlage des Strahlengangs Pupillenumriss-Hornhaut-Kamera berechnet (Ray Tracing), wobei die Ansichten von jeder der drei Kameras berücksichtigt werden.

In der Patentanmeldung WO 01/62140 wird ein System für die Messung der Topographie der beiden Hornhautoberflächen und der Hornhautdicke beschrieben. Im System gemäss WO 01/62140 wird ein Lichtstrahl, beispielsweise ein Laserstrahl, mittels einer zylinderförmigen Linse fächerförmig ausgeweitet und auf die Hornhaut des Auges gestrahlt. Das System ist so eingerichtet, dass der fächerförmige Lichtstrahl rotiert werden kann. Das beleuchtete Gebiet im Schnittbereich des fächerförmigen Lichtstrahls und der Hornhaut wird durch zwei Kameras aufgenommen, die rechtwinklig zueinander angeordnet sind, so dass ihre Beobachtungsrichtungen in der Blickrichtung der optischen Achse des Auges einen 90-Grad-Winkel einschliessen. Die durch die Kameras aufgenommen Bilder des beleuchteten Schnittbereichs sind jeweils nur dann unverzerrt, wenn die Ebene des Schnittbereichs senkrecht (in Richtung der optischen Achse des Auges gesehen) zu der Beobachtungsrichtung der betreffenden Kamera liegt. Die andere Kamera erfasst in dieser Position den Schnittbereich in Aufsicht und kann somit weder die Dicke noch das Profil der Hornhaut erfassen. Im System gemäss WO 01/62140 wird aus den Aufnahmen der beiden Kameras ein korrigiertes, unverzerrtes Bild erzeugt, wobei das unverzerrte Bild dem einer virtuellen rotierenden Kamera entspricht. Aus dem korrigierten Bild wird dann die Dicke der Hornhaut bestimmt. Durch Rotation der Lichtquelle kann die Hornhauttopographie aus mehreren korrigierten Bildern zusammengesetzt werden.

.Zur Berechnung der Hornhautdicke mittels Ray Tracing muss neben dem Brechungsindex der Hornhaut, dem Beleuchtungswinkel (Richtungen der projizierten Lichtstrahlen), dem Beobachtungswinkel (Richtung der aufgenommenen Lichtstrahlen) auch die Oberflächenneigung der Hornhaut bekannt sein. Der Brechungsindex kann als bekannt angenommen werden und sowohl der Beleuchtungs- als auch der Beobachtungswinkel kann durch Kalibrierung des Systems gemäss WO 01/62140 bestimmt werden; für die Bestimmung der Oberflächenneigung benötigt das System gemäss WO 01/62140 allerdings zusätzliche Mittel, wenn der Einfluss der Oberflächenneigung der Hornhaut das Resultat der Dickenberechnung gemäss WO 01/62140 nicht verfälschen soll.

Darstellung der Erfindung

Es ist eine Aufgabe der vorliegenden Erfindung, eine neue Vorrichtung und ein neues Verfahren zur Bestimmung von geometrischen Messwerten eines Auges vorzuschlagen, deren Ausführung einfacher als die im Stand der Technik ist und die insbesondere keine zusätzlichen, speziellen Mittel zur Bestimmung der Oberflächenneigung der Hornhaut des Auges benötigen.

Gemäss der vorliegenden Erfindung werden diese Ziele insbesondere durch die Elemente der unabhängigen Ansprüche erreicht. Weitere vorteilhafte Ausführungsformen gehen ausserdem aus den abhängigen Ansprüchen und der Beschreibung hervor.

Die Vorrichtung zur Bestimmung von geometrischen Messwerten eines Auges, insbesondere eines menschlichen Auges, umfasst einen Lichtprojektor zur Projektion eines Strahlenbündels durch einen Querschnittsteil des Auges, und Bilderfassungsmittel zur Erfassung eines ersten Abbilds von mindestens einem Teilgebiet des durch den Lichtprojektor beleuchteten Querschnittsteils unter einem ersten Beobachtungswinkel, aus einer ersten Position ausserhalb des Strahlenbündels, und zur Erfassung eines zweiten Abbilds des Teilgebiets unter einem zweiten Beobachtungswinkel, aus einer zweiten Position ausserhalb des Strahlenbündels.

Die oben genannten Ziele werden durch die Erfindung insbesondere dadurch erreicht, dass diese Vorrichtung Bildverarbeitungsmittel umfasst zur Bestimmung von Augenstrukturen aus dem erfassten ersten Abbild, zur Bestimmung von Augenstrukturen aus dem erfassten zweiten Abbild, zur Bestimmung einer ersten Distanz zwischen den aus dem ersten Abbild bestimmten Augenstrukturen, und zur Bestimmung einer zweiten Distanz zwischen den aus dem zweiten Abbild bestimmten Augenstrukturen, und dass diese Vorrichtung Verarbeitungsmittel zur Berechnung von mindestens einem der geometrischen Messwerte direkt aus der bestimmten ersten Distanz und der bestimmten zweiten Distanz umfasst.

Mit der Bestimmung der ersten und zweiten Distanz aus zwei Abbildern des beleuchteten Querschnittsteils, z.B. ein so genannter Lichtschnitt, aus zwei unabhängigen Positionen mit bekannten Beobachtungswinkeln, ergeben sich bei bekanntem Brechungsindex zwei Gleichungen mit zwei Unbekannten, die sich aus diesen Gleichungen berechnen lassen, nämlich der Oberflächenneigungswinkel der Hornhaut und die Distanz zwischen den abgebildeten Augenstrukturen entsprechenden tatsächlichen Strukturen im Auge. Der Vorteil der derart eingerichteten Vorrichtung zur Bestimmung von Distanzen zwischen Augenstrukturen und Oberflächenneigungswinkel der Hornhaut als geometrische Messwerte eines Auges besteht insbesondere in ihrer Einfachheit. Ein weiterer Vorteil dieser Vorrichtung besteht darin, dass für die Bestimmung von Distanzen zwischen Augenstrukturen keine Schätzungen oder Annahmen über den Oberflächenneigungswinkel der Hornhaut des Auges gemacht werden müssen. Damit lässt sich beispielsweise auch ein aufgeklappter Hornhautlappen, also ein Stück Hornhaut in beliebiger Lage, messen. Die Vorrichtung benötigt weder zusätzliche spezielle Mittel zur Bestimmung der Oberflächenneigung der Hornhaut noch zahlreiche Kameras oder spezielle zusätzliche Infrarotlichtquellen und sie benötigt auch keine zahlreichen Iterationsschritte, welche sowohl Zeit als auch Rechenleistung und Speicherplatz erfordern. Da die Augenstrukturen aus zwei Beobachtungswinkeln erfasst werden, kann die Bestimmung von geometrischen Messwerten auch erfolgen, wenn eines der beiden Abbilder ausnahmsweise abgeschattet ist.

In einer bevorzugten Ausführungsvariante sind die Verarbeitungsmittel der Vorrichtung eingerichtet zur Berechnung der Distanz zwischen den tatsächlichen Augenstrukturen, das heisst zwischen den Strukturen im Auge, die den aus dem ersten und aus dem zweiten Abbild bestimmten Augenstrukturen entsprechen, aus der bestimmten ersten Distanz und der bestimmten zweiten Distanz, vorzugsweise durch gewichtete Mittelwertbildung aus der bestimmten ersten Distanz und der bestimmten zweiten Distanz.

Vorzugsweise sind die Bildverarbeitungsmittel eingerichtet zur Bestimmung der Hornhaut des Auges aus dem erfassten ersten Abbild, zur Bestimmung der Hornhaut des Auges aus dem erfassten zweiten Abbild, zur Bestimmung einer ersten Distanz der aus dem ersten Abbild bestimmten Hornhaut, und zur Bestimmung einer zweiten Distanz der aus dem zweiten Abbild bestimmten Hornhaut, und die Verarbeitungsmittel sind eingerichtet zur Berechnung der Dicke der tatsächlichen Hornhaut des Auges aus der bestimmten ersten Distanz und der bestimmten zweiten Distanz. Die oben genannten Vorteile können so bei einer entsprechenden Vorrichtung zur Messung der Hornhautdicke erreicht werden.

In einer bevorzugten Ausführungsvariante sind die Verarbeitungsmittel zusätzlich oder als Alternative eingerichtet zur Berechnung eines Neigungswinkels zwischen dem Strahlenbündel und der Normalen zu der dem Lichtprojektor zugewandten Oberfläche der tatsächlichen Hornhaut des Auges aus der bestimmten ersten Distanz und der bestimmten zweiten Distanz. Die Vorrichtung kann so nicht nur zur Messung von Distanzen zwischen Augenstrukturen, insbesondere zur Messung der Hornhautdicke, sondern auch zur Messung der Oberflächenneigung der Hornhaut verwendet werden.

Vorzugsweise liegen die erste Position und die zweite Position der Bilderfassungsmittel auf verschiedenen Seiten einer durch das Strahlenbündel gelegten Ebene und der erste und der zweite Beobachtungswinkel sind gleich gross. Der Vorteil, die beiden Beobachtungswinkel gleich gross zu wählen, besteht darin, dass eine genaue Bestimmung der Oberflächenneigung der Hornhaut nicht nötig ist, da sich kleine Abweichungen von einer angenommenen oder geschätzten Oberflächenneigung nicht auf den aus der bestimmten ersten Distanz und der bestimmten zweiten Distanz berechneten Mittelwert auswirken, wenn die Vorrichtung so appliziert wird, dass das Strahlenbündel näherungsweise durch einen Meridianschnitt der Hornhaut geht. Bei der Mittelwertbildung heben sich nämlich die Abweichungen bei der Bestimmung der ersten Distanz zwischen den Augenstrukturen aus dem ersten Abbild und der zweiten Distanz zwischen den Augenstrukturen aus dem zweiten Abbild gegenseitig auf. Das heisst, die Abweichungen der aus zwei verschiedenen Perspektiven bestimmten Distanzen heben sich gegenseitig auf. Wenn folglich die Vorrichtung so angewendet wird, dass das Strahlenbündel im Wesentlichen senkrecht zu der dem Lichtprojektor zugewandten (Hornhaut-) Oberfläche des Auges projiziert wird, wirken sich geringe Verkippungen des Strahlenbündels bezüglich der Normalen zu der dem Lichtprojektor zugewandten Oberfläche der Hornhaut nicht auf die Bestimmung der Hornhautdicke aus. Auch wenn die Vorrichtung so angewendet wird, dass das Strahlenbündel im Wesentlichen senkrecht durch den Scheitelpunkt der Hornhaut projiziert wird (das heisst durch die optische Achse des Auges), wirken sich geringe Verkippungen (das heisst Neigung von der Normalen) und Exzentrizitäten (das heisst Verschiebungen vom Scheitelpunkt) des Strahlenbündels nicht auf die Bestimmung der Hornhautdicke aus. Dasselbe trifft auf kleine Abweichungen des ersten Beobachtungswinkels vom zweiten Beobachtungswinkel zu. Auch Verkippungen des Lichtprojektors wirken sich weniger kritisch auf die Messung aus. Der Vorteil gleicher Beobachtungswinkel besteht also darin, dass sich kleine Ungenauigkeiten bei der Applikation, der Justierung und/oder der Kalibrierung der Vorrichtung ohne grosse Abweichungen auf die Messresultate auswirken. Wird die Vorrichtung beispielsweise in Meridianschnitten appliziert, dann reicht eine Kalibrierung im Meridianschnitt aus, um auch bei leichten Exzentrizitäten und Verkippungen genau messen zu können. Die Vorrichtung ermöglicht so eine einfachere Applikation und Ausführung unter Beibehaltung der Genauigkeit der Messresultate.

In einer Ausführungsvariante sind die Verarbeitungsmittel eingerichtet zur Berechnung eines Verkippungsfaktors aus der bestimmten ersten Distanz und der bestimmten zweiten Distanz, welcher Verkippungsfaktor das Ausmass der Verkippung des Strahlenbündels bezüglich der Normalen zu der dem Lichtprojektor zugewandten Oberfläche der tatsächlichen Hornhaut des Auges und das Ausmass der Abweichung des ersten Beobachtungswinkels vom zweiten Beobachtungswinkel angibt. Ein solcher Verkippungsfaktor gibt ein Mass für die Güte der Applikation und der Genauigkeit der Messung. Der Verkippungsfaktor kann dem Benutzer der Vorrichtung angezeigt werden, so dass eine Korrektur der Applikation oder der Kalibrierung der Vorrichtung vorgenommen werden kann. Die Verarbeitungsmittel können auch so eingerichtet sein, dass sie in Abhängigkeit des bestimmten Verkippungsfaktors Gewichtungsfaktoren für die Mittelwertbildung bestimmen, so dass die Bestimmung der Messwerte automatisch an das Ausmass der Verkippung des Strahlenbündels, an das Ausmass der Abweichung des ersten Beobachtungswinkels vom zweiten Beobachtungswinkel und/oder an die Oberflächenneigung der Hornhaut angepasst wird.

In einer bevorzugten Ausführungsvariante umfassen die Bilderfassungsmittel einen Bildwandler, z.B. einen CCD-Chip (Charged Coupled Device) einer Kamera, und die Bilderfassungsmittel umfassen optische Elemente zur Lichtstrahlumlenkung, wobei erste der optischen Mittel so bei der ersten Position angeordnet sind, dass Lichtstrahlen zur Erzeugung des ersten Abbilds zum Bildwandler umgelenkt werden und wobei zweite der optischen Mittel so bei der zweiten Position angeordnet sind, dass Lichtstrahlen zur Erzeugung des zweiten Abbilds zum Bildwandler umgelenkt werden. Der Vorteil derart angeordneter optischer Mittel zur Lichtstrahlumlenkung besteht darin, dass das erste und das zweite Abbild, das heisst die Aufnahme zweier Perspektiven des Lichtschnitts, zeitgleich mit einer einzigen gemeinsamen Kamera erfasst werden kann. Dadurch kann auf eine zweite kostspielige Kamera und Bilderfassungshardware verzichtet werden, es erübrigt sich die Synchronisation mehrerer Bildwandler und es ergibt sich eine besonders kompakte Vorrichtung.

Vorzugsweise ist der Lichtprojektor so beschaffen, dass er das Strahlenbündel in Form einer Lichtebene projiziert. Die Lichtebene, beispielsweise in Form eines projizierten Lichtspalts, eignet sich besonders gut für die Erzeugung eines beleuchteten Lichtschnitts im Auge, der von zwei verschiedenen Positionen bildlich so erfasst werden kann, das sich entsprechende Augenstrukturen in den beiden Abbildern einander leicht zuordnen lassen.

Vorzugsweise sind die Bilderfassungsmittel in Scheimpfluganordnung zum Strahlenbündel angeordnet. Die Scheimpfluganordnung der Bilderfassungsmittel hat den Vorteil, dass entlang des Strahlenbündels über einen grossen Bereich scharf abgebildet wird.

In einer Ausführungsvariante umfasst die Vorrichtung einen Rotationstreiber zum Rotieren der Bilderfassungsmittel und des Lichtprojektors um eine durch das Strahlenbündel verlaufende Achse. Durch die Rotation der Bilderfassungsmittel und des Lichtprojektors vorzugsweise um die optische Achse des Auges können geometrische Messwerte, insbesondere die Hornhautdicke, des ganzen Auges bestimmt werden. Dabei wirken sich der hohe Grad an Symmetrie und die gleich bleibenden Messbedingungen bei der Rotation um die optische Achse des Auges positiv auf die Messgenauigkeit aus.

Kurze Beschreibung der Zeichnungen

Nachfolgend wird eine Ausführung der vorliegenden Erfindung anhand eines Beispieles beschrieben. Das Beispiel der Ausführung wird durch die folgenden beigelegten Figuren illustriert:

Figur 1 zeigt eine schematische Aufsicht des Strahlengangs eines Strahlenbündels, das von einem Lichtprojektor durch die Hornhaut eines Auges projiziert und durch Bilderfassungsmittel erfasst wird.

Figur 2 zeigt eine schematische Aufsicht einer Vorrichtungsanordnung, welche einen Lichtprojektor zur Projektion eines Strahlenbündels umfasst und welche zwei Bilderfassungsvorrichtungen zur Erfassung des durch den Lichtprojektor beleuchteten Querschnittsteils der Hornhaut aus zwei Beobachtungspositionen umfasst.

Figur 3 zeigt eine schematische Aufsicht einer Vorrichtung zur Bestimmung von geometrischen Messwerten eines Auges, welche ein Strahlenbündel durch die Hornhaut des Auges projiziert und welche den durch das Strahlenbündel beleuchteten Querschnittsteils der Hornhaut aus zwei Beobachtungspositionen erfasst.

Figur 4 zeigt ein Doppelbild mit einem ersten und einem zweiten Abbild des beleuchteten Querschnittsteils der Hornhaut eines Auges.

Wege zur Ausführung der Erfindung

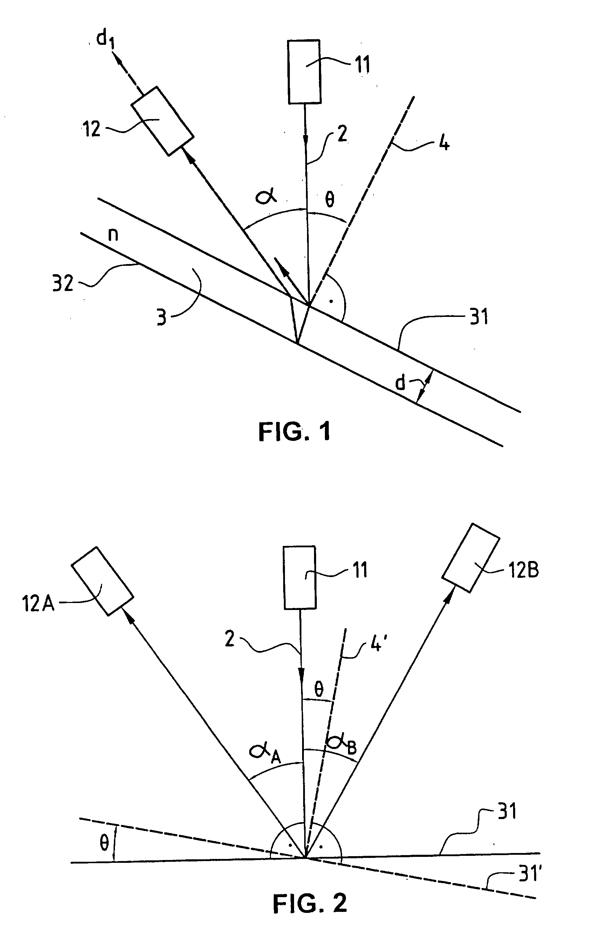

In der nachfolgenden Beschreibung ist bei der Bezugnahme auf die Figuren 1 bis 3 zu beachten, dass Überlegungen, die diese Figuren betreffen, exemplarisch für die Zeichenebene gemacht werden, dass jedoch Überlegungen für Ebenen parallel zu der Zeichenebene entsprechend gelten. Der Begriff senkrecht bezieht sich auf die Zeichenebene. Bei Lichtstrahlen, die nicht in der Ebene eines Meridians der Hornhaut liegen, gibt es eine zusätzliche Strahlkomponente, die aus der Zeichenebene herauszeigt. Hierdurch ändern sich je nach optischem Aufbau die funktionalen Zusammenhänge der Bildentstehung. Da dies keine Auswirkungen auf die oben genannten Vorteile des Verfahrens hat, wird im folgenden nicht mehr gesondert darauf eingegangen.

In der Figur bezieht sich das Bezugszeichen 3 auf eine vereinfachte schematische Darstellung eines optisch streuenden Körpers, insbesondere eine Hornhaut, mit dem Brechungsindex n und der Dicke d. In der Figur 1 projiziert ein Lichtprojektor 11 ein Strahlenbündel 2 durch die Hornhaut 3. Entsprechend der Struktur des Strahlenbündels 2 wird ein Querschnittsteil der Hornhaut 3 beleuchtet. Es sei an dieser Stelle bemerkt, dass das Strahlenbündel 2 in der Praxis eine räumliche Struktur aufweist, die in der schematischen Darstellung der Figur 1 nicht wiedergegeben ist. Mit dem Bezugszeichen 4 wird eine Oberflächennormale zur Oberfläche 31 der Hornhaut 3 bezeichnet. Der Einfallswinkel $\ominus$ gibt den Winkel zwischen dem Strahlenbündel 2 und der Oberflächennormalen 4 an. Das Bezugszeichen 12 bezeichnet eine Bilderfassungsvorrichtung mit Verarbeitungsmitteln, welche den in der Hornhaut 3 beleuchteten Quer-schnittsteil unter einem Beobachtungswinkel $\alpha$ erfasst. Gemäss dem bekannten Snelliusschen Brechungsgesetz hängen die in einem transparenten Körper, hier die Hornhaut 3, zurückgelegten Weglängen der Lichtstrahlen von den Brechungsindizes des transparenten Körpers und des umgebenden Mediums sowie vom Einfallswinkel und vom Ausfallswinkel der Lichtstrahlen ab. Wird aus dem Abbild des durch den Lichtprojektor 11 beleuchteten Querschnittsteils der Hornhaut 3, das durch die Bilderfassungsvorrichtung 12 aufgenommen wird, die Distanz $d_1$ zwischen der abgebil-deten vorderen Hornhautoberfläche 31 und der abgebildeten hinteren Hornhautoberfläche 32 bestimmt, kann die Dicke d der tatsächlichen Hornhaut 3 folglich gemäss analytisch oder experimentell gewonnenen Gleichungen (i) als Funktion dieser bestimmten Distanz $d_1$, des Einfallswinkels $\Theta$, des Beobachtungswinkels $\alpha$ und des Brechungsindex n berechnet werden (der Einfluss der Dicke des Strahlenbündels wurde hier nicht angeführt; die Gleichung kann auch in implizierter Form vorliegen):

$$d = f(d_1, \ominus, \alpha, n) \qquad\qquad (i)$$

Im Unterschied zu der in der Figur 1 dargestellten Vorrichtungsanordnung, umfasst die in der Figur 2 dargestellte Vorrichtungsanordnung zwei Bilderfassungsvorrichtungen 12A und 12B, beispielsweise CCD-Kameras (Charged Coupled Device) oder CMOS-Kameras (Complementary Metal-Oxide-Silicon). Durch die Bilderfassungsvorrichtung 12A wird der durch den Lichtprojektor 11 beleuchtete Querschnittsteil der Hornhaut unter dem Beobachtungs-winkel $\alpha_A$ erfasst und abgebildet. Durch die Bilderfassungsvorrichtung 12B wird der durch den Lichtprojektor 11 beleuchtete Querschnittsteil der Hornhaut unter dem Beobachtungswinkel $\alpha_B$ erfasst und abgebildet. Der Lichtprojektor 11 projiziert das Strahlenbündel 2 vorzugsweise,in Form einer Lichtebene, insbesondere als projizierter Lichtspalt, so dass sich als beleuchteter Querschnittsteil ein Lichtschnitt ergibt. Das Strahlenbündel 2 kann jedoch auch eine strahlförmige Struktur haben. Der Lichtprojektor 11 umfasst beispielsweise eine Spaltlampe oder einen Laser, dessen Licht durch Strahlumformungsoptiken zu einem Fächer geformt wird. Die Beobachtungswinkel $\alpha_A$ und $\alpha_B$ können unterschiedlich sein und sich auch auf der gleichen Seite des Strahlenbündels befinden. Die beiden Bilderfassungsvorrichtungen 12A und 12B sind jedoch vorzugsweise in einer gemeinsamen Ebene senkrecht zu der Lichtebene positioniert. Wie in der Figur 2 schematisch dargestellt ist, wird das Strahlenbündel 2 des Lichtprojektors 11 senkrecht (bezogen auf die Zeichenebene) zur Hornhautoberfläche 31 durch die Hornhaut projiziert. Weist die Hornhautoberfläche 31' jedoch eine durch die gestrichelte Linie dargestellte Neigung mit dem Oberflächenneigungswinkel $\Theta$ auf, so ist das Strahlenbündel 2 um den entsprechenden Einfallswinkel $\Theta$ von der Oberflächennormalen 4' zur geneigten Hornhautoberfläche 31' verkippt.

Aus den beiden Abbildern des beleuchteten Querschnittsteils der Hornhaut aus zwei verschiedenen Positionen lassen sich die Distanz $d_A$, zwischen der vorderen und hinteren Hornhautoberfläche aus dem Abbild der Bilderfassungsvorrichtung 12A, und die Distanz $d_B$, zwischen der vorderen und hinteren Hornhautoberfläche aus dem Abbild der Bilderfassungsvorrichtung 12B, bestimmen. Da die Beobachtungswinkel $\alpha_A$ und $\alpha_B$ aus der Geometrie der Vorrichtungsanordnung bekannt sind und da der Brechungsindex n ebenfalls bekannt ist, ergeben sich zwei Gleichungen (i) mit zwei Unbekannten zur Berechnung der Dicke d der tatsächlichen Hornhaut und des Einfallswinkels $\ominus$, der dem Oberflächenneigungswinkel $\ominus$ der Hornhaut entspricht.

.Vorzugsweise werden die beiden Bilderfassungsvorrichtungen 12A und 12B so angeordnet, dass die Beobachtungswinkel $\alpha_A$ und $\alpha_B$ den gleichen Betrag haben und dass näherungsweise senkrecht zur Hornhautoberfläche 31 projiziert wird. Die Konfiguration mit gleichen Beobachtungswinkeln $\alpha_A$ und $\alpha_B$ ermöglicht eine genaue Bestimmung der Dicke d der Hornhaut 3, ohne den Oberflächenneigungswinkel $\ominus$ respektive den Einfallswinkel $\ominus$ genau bestimmen zu müssen. Stellt man für die bestimmten Distanzen $d_A$ und $d_B$ eine Taylorreihe gemäss den Gleichungen ($ii_A$, $ii_B$) auf:

$$d_A = d_{A(\theta=0)} + \frac{\partial d_A}{\partial \theta} \, d\theta + \frac{1}{2} \frac{\partial^2 d_A}{\partial \theta^2} \, d\theta^2 + ... \qquad \text{(iiA)}$$

$$d_B = d_{B(\theta=0)} - \frac{\partial d_B}{\partial \theta} \, d\theta + \frac{1}{2} \frac{\partial d_B}{\partial \theta^2} \, d\theta^2 - ... \qquad \text{(iiB)}$$

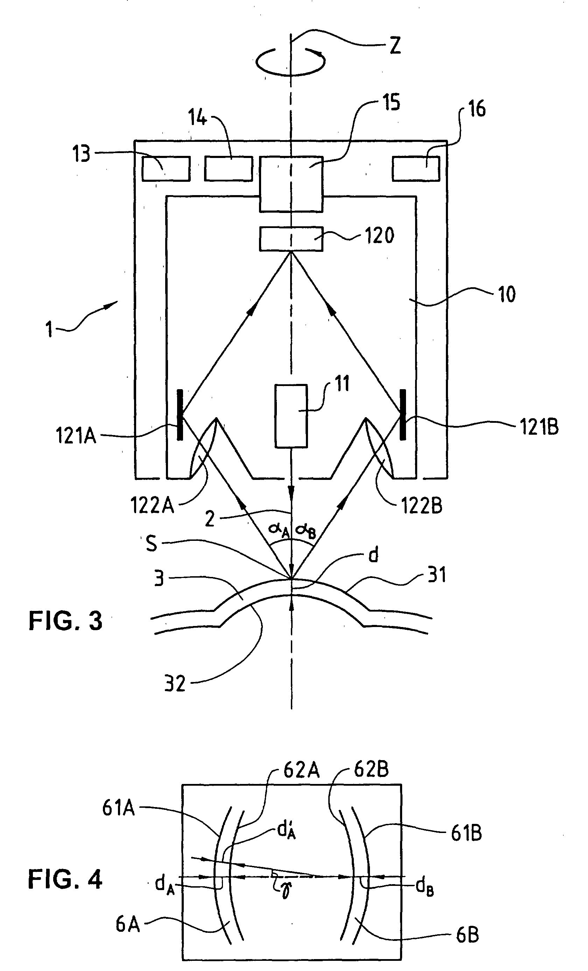

so ist es zulässig, die Reihe für kleine Abweichungen vom Oberflächenneigungswinkel $\Theta$ respektive Einfallswinkel $\Theta$ nach dem zweiten Glied abzubrechen. Beim Berechnen des arithmetischen Mittels aus den dementsprechend reduzierten Taylorreihen für die bestimmte Distanz $d_A$ und für die bestimmte Distanz $d_B$ wirkt sich eine Abweichung $d\Theta$ nicht aus, da eine solche Abweichung aus den unterschiedlichen Positionen der beiden Bilderfassungsvorrichtungen 12A und 12B jeweils als Winkel mit unterschiedlichem Vorzeichen erfasst wird und sich Abweichungen somit aufheben. Wenn zum Beispiel bei einer geplanten senkrechten Projektion des Strahlenbündels 2 durch den Scheitelpunkt S der Hornhaut (siehe Figur 3) bei der tatsächlichen Applikation eine seitliche Verschiebung vom Scheitelpunkt S (das heisst eine Exzentrizität) von einem Millimeter gemacht wird, werden die durch den entsprechenden Oberflächenneigungswinkel $\Theta$ am verschobenen Messpunkt verursachten Messabweichungen, im Vergleich zu einer Messung mit nur einer Bilderfassungsvorrichtung 12 aus einer Position, um mindestens einen Faktor zehn reduziert. Für einen Beobachtungswinkel von 45 Grad werden so die Messabweichungen bei Exzentrizität bis zu einem Millimeter (bzw. bei entsprechenden Verkippungen) deutlich unter fünf Mikrometer gehalten.

In der Figur 3 bezeichnet das Bezugszeichen 1 eine bevorzugte Ausführung der Vorrichtung zur Bestimmung von geometrischen Messwerten eines Auges. Die mit gleichen Bezugszeichen versehenen Elemente in der Figur 3 entsprechen denjenigen der Vorrichtungsanordnung der Figur 2. In der Figur 3 wird die Vorrichtung 1 so appliziert, dass das Strahlenbündel 2 vom Lichtprojektor 11 im Wesentlichen senkrecht zur vorderen Hornhautoberfläche 31 durch den Scheitelpunkt S der Hornhaut 3 projiziert wird. Wie in der Vorrichtungsanordnung gemäss der Figur 2 wird der durch das Strahlenbündel 2 beleuchtete Querschnittsteil der Hornhaut 3 durch die Vorrichtung 1 aus zwei unterschiedlichen Positionen erfasst und abgebildet. Allerdings unterscheiden sich die Bilderfassungsmittel der Vorrichtung 1 von denjenigen in der Vorrichtungsanordnung der Figur 2 dadurch, dass sie nur einen (einzigen) gemeinsamen Bildwandler 120 umfassen. Die Bilderfassungsmittel der Vorrichtung 1 umfassen zudem strahlabbildende optische Elemente 122A und 122B, zum Beispiel Objektive oder Linsen, und strahlumlenkende optische Elemente 121A und 121B, zum Beispiel Spiegel. Wie in der Figur 3 schematisch dargestellt ist, sind jeweils ein strahlabbildendes Element 122A respektive 122B und ein strahlumlenkendes Element 121A respektive 121B paarweise so angeordnet, dass Lichtstrahlen des beleuchteten Querschnittsteils der Hornhaut 3 mit einem Beobachtungswinkel $\alpha_A$ respektive $\alpha_B$ durch die strahlabbildenden Elemente 122A respektive 122B in Richtung der strahlumlenkenden Elemente 121A respektive 121B abgebildet werden, und dass diese Lichtstrahlen durch die strahlumlenkenden Elemente 121A respektive 121B zum gemeinsamen Bildwandler 120 umgelenkt werden. Die Beobachtungswinkel $\alpha_A$ und $\alpha_B$ haben vorzugsweise den gleichen Betrag. Wie in der Figur 3 schematisch dargestellt ist, sind die strahlabbildende Elemente 122A und 122B und die strahlumlenkenden Elemente 121A und 121B vorzugsweise symmetrisch (zu der optischen Achse Z des Auges) in einer Ebene senkrecht zu der Lichtebene angeordnet. Der Bildwandler 120, beispielsweise ein CCD-Chip, ist vorzugsweise in Scheimpfluganordnung zum Strahlenbündel 2 angeordnet. Der Fachmann wird verstehen, dass die in der Figur 3 dargestellte Anordnung der optischen Elemente nur eine von vielen möglichen Anordnungen ist, um zwei Abbilder von mindestens einem Teilgebiet des durch den Lichtprojektor 11 beleuchteten Querschnittsteils der Hornhaut 3 unter zwei Beobachtungswinkeln $\alpha_A$ und $\alpha_B$ auf einem gemeinsamen Bildwandler 120 zu erzeugen. Es wäre beispielsweise auch möglich, Spiegel vor den Linsen anzuordnen, weitere Spiegel und Linsen einzusetzen, Spiegeloptiken zu verwenden und/oder die optischen Elemente asymmetrisch anzuordnen sowie anamorphotische Abbildungen zu verwenden.

Im gemeinsamen Bildwandler 120 wird ein Doppelbild mit einem ersten unter dem Beobachtungswinkel $\alpha_A$ aufgenommenen Abbild 6A des beleuchteten Querschnittsteils der Hornhaut 3 und einem zweiten unter dem Beobachtungswinkel $\alpha_B$ aufgenommenen Abbild 6B des beleuchteten Querschnittsteils der Hornhaut 3 erzeugt, wie in der Figur 4 schematisch für einen Lichtschnitt dargestellt wird. In der Figur 4 ist mit $d_A'$ angedeutet, dass die Dicke der Hornhaut auf Grund der Gesetzmässigkeiten der Bildentstehung sich zum Rand scheinbar verjüngen kann. Diese scheinbare Veränderung der Dicke lässt sich bei der Berechnung der tatsächlichen Dicke der Hornhaut 3 berücksichtigen, indem beispielsweise der Winkel $\gamma$ zur Symmetrieebene des Lichtschnitts bestimmt wird. Eine Korrekturfunktion in Abhängigkeit vom Winkel $\gamma$ kann dann die bestimmten Dickenwerte korrigieren. Damit lässt sich auf einfache Weise eine Kalibrierung, die streng genommen nur für den Scheitelpunkt S gültig ist, auch auf seitliche Bereiche der Hornhaut ausdehnen. Es sei angemerkt, dass sich bei einem anders gewählten Arbeitsabstand die beleuchteten Querschnittsteile im Doppelbild auch überlagern können (wie in Figur 3 für den Scheitelpunkt S angedeutet). Lassen sich die Teilbilder nicht über Methoden der Bildverarbeitung trennen, so kann alternativ über Filter (z.B. Farbfilter bei gleichzeitiger Verwendung einer Farbkamera) oder Shuttern eine Bildtrennung auf optischem Weg erfolgen.

Die Vorrichtung 1 umfasst Bildverarbeitungsmittel 13 mit programmierten Softwaremodulen, welche einen Prozessor der Vorrichtung 1 so steuern, dass dieser die nachfolgend beschriebenen Bildverarbeitungsfunktionen ausführt. Die Bildverarbeitungsmittel 13 bestimmen jeweils aus dem erfassten ersten Abbild 6A und dem erfassten zweiten Abbild 6B definierte Augenstrukturen wie die Hornhaut, die durch das Abbild der vorderen Hornhautoberfläche 61A respektive 61B und das Abbild der hinteren Hornhautoberfläche 62A respektive 62B bestimmt ist, oder die Umrisse der Iris und der Pupille (nicht dargestellt), oder andere Merkmale wie die Vorderkammertiefe oder der Vorderkammerwinkel (nicht dargestellt). Nachfolgend bestimmen die Bildverarbeitungsmittel 13 definierte Distanzen zwischen den bestimmten abgebildeten Augenstrukturen, wie die Hornhautdicke, das heisst die Distanz $d_A$ zwischen dem Abbild der vorderen Hornhautoberfläche 61A und dem Abbild der hinteren Hornhautoberfläche 62A respektive die Distanz $d_B$ zwischen dem Abbild der vorderen Hornhautoberfläche 61B und dem Abbild der hinteren Hornhautoberfläche 62B. Figur 4 zeigt eine bevorzugte Ausführung, in der die Distanzen direkt im Abbild der Hornhaut bestimmt werden.

Die Vorrichtung 1 umfasst Verarbeitungsmittel 14 mit programmierten Softwaremodulen, welche einen Prozessor der Vorrichtung 1 so steuern, dass dieser die nachfolgend beschriebenen Funktionen ausführt. Die Verarbeitungsmittel 14 berechnen aus den durch die Bildverarbeitungsmittel 13 bestimmten Distanzen geometrische Messwerte des Auges, wie Distanzen zwischen den tatsächlichen Strukturen des Auges, insbesondere die Hornhautdicke d, oder den Oberflächenneigungswinkel $\Theta$ der Hornhaut 3. Insbesondere berechnen die Verarbeitungsmittel 14 aus der Distanz $d_A$ und der Distanz $d_B$ durch gewichtete Mittelwertbildung die Hornhautdicke d. Die Gewichtungsfaktoren für die Mittelwertbildung ergeben sich aus der Gleichung (i) bei bekanntem Brechungsindex n und bei bekannten Beobachtungswinkeln. Beispielsweise ergibt sich bei $\alpha_A = \alpha_B = \alpha$, und $\ominus = 0$ für den Fall der Parallelperspektive in erster Näherung der Gewichtungsfaktor c aus der Gleichung (iii):

$$c = \frac{\sqrt{n^2 - \sin(\alpha)^2}}{\cos(\alpha)\sin(\alpha)} \qquad (iii)$$

Auf Grund der Symmetrie der Anordnung sind die Gewichtungsfaktoren für $d_A$ und $d_B$ in diesem Fall gleich gross und die Hornhautdicke d ergibt sich dann durch gewichtete Mittelwertbildung gemäss der Gleichung (iv):

$$d = c \, \frac{(d_B + d_A)}{2} \qquad (iv)$$

Die Verarbeitungsmittel 14 können aber auch so beschaffen sein, dass sie aus der Distanz $d_A$ und der Distanz $d_B$ den Oberflächenneigungswinkel $\ominus$ der Hornhaut 3 berechnen, der wie bereits erwähnt dem Einfallswinkel $\ominus$ zwi-

schen dem Strahlenbündel 2 und einer Oberflächennormale 4 zur vorderen Hornhautoberfläche 31 entspricht. Der Oberflächenneigungswinkel $\ominus$ ergibt sich im oberen Beispiel bei bekanntem Brechungsindex n und bei bekannten Beobachtungswinkeln $\alpha_A = \alpha_B = \alpha$ für die Parallelperspektive aus der Gleichung (v):

$$\theta = \frac{\sin(\alpha)n}{\sqrt{n^2 - \sin(\alpha)^2}} \frac{(d_B - d_A)}{(d_B + d_A)} \tag{v}$$

Die Verarbeitungsmittel 14 berechnen aus der Distanz $d_A$ und der Distanz $d_B$ auch einen Verkippungsfaktor k gemäss der Gleichung (vi), der das Ausmass der Verkippung des Strahlenbündels 2 bezüglich der Oberflächennormalen 4 zu der vorderen Hornhautoberfläche 31 (respektive das Ausmass der Exzentrizität) und/oder das Ausmass der Abweichung des Beobachtungswinkels $\alpha_A$ vom Beobachtungswinkel $\alpha_B$ angibt.

$$k = \frac{d_B - d_A}{d_B + d_A} \tag{vi}$$

Solange der Verkippungsfaktor k innerhalb eines definierten Toleranzbereichs liegt, verwenden die Verarbeitungsmittel 14 die gewichtete Mittelwertbildung zur Berechnung der Hornhautdicke d ohne die Anpassung der Gewichtungsfaktoren an die Verkippung.

In einer Ausführungsvariante lassen sich die Gewichtungsfaktoren als Funktion des Verkippungsfaktors anpassen.

In einer Ausführungsvariante zeigen die Verarbeitungsmittel 14 den Verkippungsfaktor k als Applikationshilfe für den Benutzer der Vorrichtung 1 auf der Anzeige 16 an.

Der Verkippungsfaktor k kann durch die Verarbeitungsmittel 14 auch bei der Berechnung der Hornhautdicke d als Entscheidungsgrundlage für die automatische Umschaltung von einem ersten Berechnungsmodus mit gewichteter Mittelwertbildung zu einem zweiten Berechnungsmodus verwendet werden, in welchem zusätzlich zur Hornhautdicke d auch der Einfallswinkel $\Theta$ respektive der Oberflächenneigungswinkel $\Theta$ der Hornhaut als unbekannt angenommen wird und zwei Gleichungen (i) für die beiden Bilderfassungsvorrichtungen 12A und 12B, respektive für die entsprechenden Bilderfassungsmittel 120, 121A, 121B, 122A, 122B, gelöst werden.

Die Verarbeitungsmittel 14 können die Dicke der Hornhaut 3 und den Oberflächenneigungswinkel $\ominus$ für sämtliche Punkte der vorderen Hornhautoberfläche 31 berechnen, die im erfassten Teilgebiet des beleuchteten Querschnittsteils der Hornhaut 3 liegt.

In der Vorrichtung 1 sind der Bildwandler 120, die strahlabbildenden Elemente 122A und 122B, die strahlumlenkenden Elemente 121A und 121B sowie der Lichtprojektor 11 in einer drehbaren Trägervorrichtung 10 angebracht, die durch einen Rotationstreiber 15, beispielsweise ein Elektromotor, um die optische Achse Z des Auges rotiert wird. Durch die Rotation des Bildprojektors 11 und der Bilderfassungsmittel 120, 121A, 121B, 122A und 122B um die optische Achse Z wird die gesamte Hornhaut 3 vermessen. In dieser Konfiguration lassen sich auf Grund der hohen Symmetrie die geringsten Messunsicherheiten erzielen.

Die Bildverarbeitungsmittel 13 und die Verarbeitungsmittel 14 umfassen programmierte Softwaremodule, die auf einem gemeinsamen Prozessor oder auf mehreren Prozessoren ausgeführt werden. Für die Zwecke des vorliegenden Texts ist die Unterscheidung von Bildverarbeitungsmitteln 13 und Verarbeitungsmitteln 14 rein begrifflicher Natur, das heisst Funktionen die den Bildverarbeitungsmitteln 13 zugeordnet werden, könnten auch den Verarbeitungsmitteln 14 zugeordnet werden und umgekehrt (wie z.B. die Bestimmung von Distanzen). Der Fachmann wird verstehen, dass die Funktionen der Softwaremodule der Bildverarbeitungsmittel 13 und der Verarbeitungsmittel 14 auch hardwaremässig ausgeführt werden können.

An dieser Stelle soll angemerkt werden, dass die Vorrichtung 1 vorzugsweise als kompakte Messprobe ausgeführt ist, wobei die für die flächendeckende Berechnung der Hornhautdicke d und des Oberflächenneigungswinkels $\ominus$ zuständigen Module der Verarbeitungsmittel 14 in einer externen Verarbeitungseinheit, beispielsweise in einem Personal Computer, ausgeführt sein können, wobei der Datenaustausch mit der Vorrichtung 1 über eine kontaktbehaftete oder kontaktlose Kommunikationsverbindung erfolgt. Berechnete Werte der Hornhautdicke d und des Oberflächenneigungswinkels $\ominus$ können auf der Anzeige 16 oder auf einer Anzeige der externen Verarbeitungseinheit angezeigt werden.

Es sei hier zudem bemerkt, dass die Vorrichtung 1 auch auf die Projektion mehrerer Strahlenbündel erweitert werden kann. Zudem könnten auch Abbilder aus mehr als zwei Beobachtungswinkeln erfasst werden, allerdings ist dies nur dann sinnvoll, wenn zusätzliche Parameter, wie zum Beispiel die Breite des Strahlenbündels, erfasst werden sollen (drei Gleichungen mit drei Unbekannten). Zum besseren Verständnis soll auch bemerkt werden, dass der in der Beschreibung verwendete Beobachtungswinkel $\alpha$ ($\alpha_A$, $\alpha_B$) repräsentativ für einen Beobachtungsstrahl steht. Werden zentralperspektivische Abbildungen verwendet so variiert $\alpha$.

Abschliessend sei bemerkt, dass die oben angeführten Überlegungen zur Bestimmung der Hornhautdicke entsprechend auch auf die Bestimmung anderer Distanzen anwendbar sind.

**Legende der Bezugszeichen**

| | |
|---|---|
| 1 | Vorrichtung zur Bestimmung von geometrischen Messwerten eines Auges |
| 2 | Strahlenbündel |
| 3 | Hornhaut |
| 4 | Normale |
| 6A | erstes Abbild |
| 6B | zweites Abbild |
| 10 | drehbare Trägervorrichtung |
| 11 | Lichtprojektor |
| 12 | Bilderfassungsvorrichtung mit Verarbeitungsmitteln |
| 12A, 12B | Bilderfassungsvorrichtung |
| 13 | Bildverarbeitungsmittel |
| 14 | Verarbeitungsmittel |
| 15 | Rotationstreiber |
| 16 | Anzeige |
| 31, 31' | vordere Hornhautoberfläche |
| 32 | hintere Hornhautoberfläche |
| 61A, 61B | Abbild der vorderen Hornhautoberfläche |
| 62A, 62B | Abbild der hinteren Hornhautoberfläche |
| 120 | Bildwandler |
| 121A, 121B | Strahlumlenkende optische Elemente (Spiegel) |
| 122A, 122B | Strahlabbildende optische Elemente (Linsen) |
| $\alpha$, $\alpha_A$, $\alpha_B$ | Beobachtungswinkel |
| d | Dicke (Distanz) |
| $d_A$ | erste Distanz |
| $d_B$ | zweite Distanz |
| $d_1$ | Distanz |
| n | Brechungsindex |
| $\ominus$ | Einfallswinkel (entspricht Oberflächenneigungswinkel) |
| S | Scheitelpunkt der Hornhaut |
| Z | optische Achse eines Auges |

**Patentansprüche**

1. Vorrichtung (1) zur Bestimmung von geometrischen Messwerten (d) eines Auges, insbesondere eines menschlichen Auges, umfassend:

   - einen Lichtprojektor (11) zur Projektion eines Strahlenbündels (2) durch einen Querschnittteil des Auges, und

   - Bilderfassungsmittel (120, 121A, 121B, 122A, 122B) zur Erfassung eines ersten Abbilds (6A) von mindestens einem Teilgebiet des durch den Lichtprojektor (11) beleuchteten Querschnittsteils unter einem ersten Beobachtungswinkel ($\alpha_A$), aus einer ersten Position ausserhalb des Strahlenbündels (2), und zur Erfassung eines zweiten Abbilds (6B) des Teilgebiets unter einem zweiten Beobachtungswinkel ($\alpha_B$), aus einer zweiten Position ausserhalb des Strahlenbündels (2),

     **gekennzeichnet durch**:

   - Bildverarbeitungsmittel (13) zur Bestimmung von Augenstrukturen aus dem erfassten ersten Abbild (6A), zur Bestimmung von Augenstrukturen aus dem erfassten zweiten Abbild (6B), zur Bestimmung einer ersten Distanz ($d_A$) zwischen den aus dem ersten Abbild (6A) bestimmten Augenstrukturen, und zur Bestimmung einer zweiten Distanz ($d_B$) zwischen den aus dem zweiten Abbild (6B) bestimmten Augenstrukturen, und

- Verarbeitungsmittel (14) zur Berechnung von mindestens einem der geometrischen Messwerte (d) aus der bestimmten ersten Distanz ($d_A$) und der bestimmten zweiten Distanz ($d_B$).

2. Vorrichtung (1) gemäss Anspruch 1, **dadurch gekennzeichnet, dass** die Verarbeitungsmittel (14) eingerichtet sind zur Berechnung einer Distanz (d) zwischen den tatsächlichen Augenstrukturen (31, 32) aus der bestimmten ersten Distanz ($d_A$) und der bestimmten zweiten Distanz ($d_B$).

3. Vorrichtung (1) gemäss Anspruch 2, **dadurch gekennzeichnet, dass** die Verarbeitungsmittel (14) eingerichtet sind zur Berechnung der Distanz (d) zwischen den tatsächlichen Augenstrukturen (31, 32) durch gewichtete Mittelwertbildung aus der bestimmten ersten Distanz ($d_A$) und der bestimmten zweiten Distanz ($d_B$).

4. Vorrichtung (1) gemäss Anspruch 1, **dadurch gekennzeichnet, dass** die Bildverarbeitungsmittel (13) eingerichtet sind zur Bestimmung der Hornhaut des Auges aus dem erfassten ersten Abbild (6A), zur Bestimmung der Hornhaut des Auges aus dem erfassten zweiten Abbild (6B), zur Bestimmung einer ersten Distanz ($d_A$) der aus dem ersten Abbild (6A) bestimmten Hornhaut und zur Bestimmung einer zweiten Distanz ($d_B$) der aus dem zweiten Abbild (6B) bestimmten Hornhaut, und dass die Verarbeitungsmittel (14) eingerichtet sind zur Berechnung der Dicke (d) der tatsächlichen Hornhaut (3) des Auges aus der bestimmten ersten Distanz ($d_A$) und der bestimmten zweiten Distanz ($d_B$).

5. Vorrichtung (1) gemäss Anspruch 1, **dadurch gekennzeichnet, dass** die Bildverarbeitungsmittel (13) eingerichtet sind zur Bestimmung der Hornhaut des Auges aus dem erfassten ersten Abbild (6A), zur Bestimmung der Hornhaut des Auges aus dem erfassten zweiten Abbild (6B), zur Bestimmung einer ersten Distanz ($d_A$) der aus dem ersten Abbild (6A) bestimmten Hornhaut, und zur Bestimmung einer zweiten Distanz ($d_B$) der aus dem zweiten Abbild (6B) bestimmten Hornhaut, und dass die Verarbeitungsmittel (14) eingerichtet sind zur Berechnung eines Neigungswinkels zwischen dem Strahlenbündel (2) und einer Normalen zu der dem Lichtprojektor (11) zugewandten Oberfläche der tatsächlichen Hornhaut des Auges aus der bestimmten ersten Distanz ($d_A$) und der bestimmten zweiten Distanz ($d_B$).

6. Vorrichtung (1) gemäss Anspruch 1, **dadurch gekennzeichnet, dass** die erste Position und die zweite Position auf verschiedenen Seiten einer durch das Strahlenbündel (2) gelegten Ebene liegen und dass der erste ($\alpha_A$) und der zweite Beobachtungswinkel ($\alpha_B$) gleich gross sind.

7. Vorrichtung (1) gemäss Anspruch 1, **dadurch gekennzeichnet, dass** die Bildverarbeitungsmittel (13) eingerichtet sind zur Bestimmung der Hornhaut des Auges aus dem erfassten ersten Abbild (6A), zur Bestimmung der Hornhaut des Auges aus dem erfassten zweiten Abbild (6B), zur Bestimmung einer ersten Distanz ($d_A$) der aus dem ersten Abbild (6A) bestimmten Hornhaut, und zur Bestimmung einer zweiten Distanz ($d_B$) der aus dem zweiten Abbild (6B) bestimmten Hornhaut, und dass die Verarbeitungsmittel (14) eingerichtet sind zur Berechnung eines Verkippungsfaktors aus der bestimmten ersten Distanz ($d_A$) und der bestimmten zweiten Distanz ($d_B$), welcher Verkippungsfaktor das Ausmass der Verkippung des Strahlenbündels (2) bezüglich der Normalen zu der dem Lichtprojektor (11) zugewandten Oberfläche (31) der tatsächlichen Hornhaut (3) des Auges und das Ausmass der Abweichung des ersten Beobachtungswinkels ($\alpha_A$) vom zweiten Beobachtungswinkel ($\alpha_B$) angibt.

8. Vorrichtung (1) gemäss Anspruch 1, **dadurch gekennzeichnet, dass** die Bildverarbeitungsmittel (13) eingerichtet sind zur Bestimmung der Hornhaut des Auges aus dem erfassten ersten Abbild (6A), zur Bestimmung der Hornhaut des Auges aus dem erfassten zweiten Abbild (6B), zur Bestimmung einer ersten Distanz ($d_A$) der aus dem ersten Abbild (6A) bestimmten Hornhaut, und zur Bestimmung einer zweiten Distanz ($d_B$) der aus dem zweiten Abbild (6B) bestimmten Hornhaut, dass die Verarbeitungsmittel (14) eingerichtet sind zur Berechnung der Dicke (d) der tatsächlichen Hornhaut (3) des Auges durch gewichtete Mittelwertbildung aus der bestimmten ersten Distanz ($d_A$) und der bestimmten zweiten Distanz ($d_B$), dass die Verarbeitungsmittel (14) eingerichtet sind zur Berechnung eines Verkippungsfaktors aus der bestimmten ersten Distanz ($d_A$) und der bestimmten zweiten Distanz ($d_B$), welcher Verkippungsfaktor das Ausmass der Verkippung des Strahlenbündels (2) bezüglich der Normalen zu der dem Lichtprojektor (11) zugewandten Oberfläche (31) der tatsächlichen Hornhaut (3) des Auges und das Ausmass der Abweichung des ersten Beobachtungswinkels ($\alpha_A$) vom zweiten Beobachtungswinkel ($\alpha_B$) angibt, und dass die Verarbeitungsmittel (14) eingerichtet sind zur Bestimmung von Gewichtungsfaktoren für die Mittelwertbildung in Abhängigkeit des bestimmten Verkippungsfaktors.

9. Vorrichtung (1) gemäss Anspruch 1, **dadurch gekennzeichnet, dass** die Bilderfassungsmittel einen Bildwandler (120) umfassen und dass die Bilderfassungsmittel optische Elemente zur Lichtstrahlumlenkung (121A, 121B) umfassen, wobei erste der optischen Mittel (121A) so bei der ersten Position angeordnet sind, dass Lichtstrahlen zur Erzeugung des ersten Abbilds (6A) zum Bildwandler (120) umgelenkt werden und wobei zweite der optischen Mittel (121B) so bei der zweiten Position angeordnet sind, dass Lichtstrahlen zur Erzeugung des zweiten Abbilds (6B) zum Bildwandler (120) umgelenkt werden.

10. Vorrichtung (1) gemäss Anspruch 1, **dadurch gekennzeichnet, dass** der Lichtprojektor (11) so beschaffen ist, dass er das Strahlenbündel (2) in Form einer Lichtebene projiziert.

11. Vorrichtung (1) gemäss Anspruch 1, **dadurch gekennzeichnet, dass** die Bilderfassungsmittel (120, 121A, 121B, 122A, 122B) in Scheimpfluganordnung zum Strahlenbündel (2) angeordnet sind.

12. Vorrichtung (1) gemäss Anspruch 1, **dadurch gekennzeichnet, dass** sie einen Rotationstreiber (15) umfasst zum Rotieren des Lichtprojektors (11) und der Bilderfassungsmittel (120, 121A, 121B, 122A, 122B) um eine durch das Strahlenbündel (2) verlaufende Achse (Z).

13. Verfahren zur Bestimmung von geometrischen Messwerten (d) eines Auges, insbesondere eines menschlichen Auges, in welchem Verfahren mittels eines Lichtprojektors (11) ein Strahlenbündel (2) durch einen Querschnittsteil des Auges projiziert wird, in welchem Verfahren ein erstes Abbild von mindestens einem Teilgebiet des durch den Lichtprojektor (11) beleuchteten Querschnittsteils unter einem ersten Beobachtungswinkel ($\alpha_A$), aus einer ersten Position ausserhalb des Strahlenbündels (2) erfasst wird, in welchem Verfahren ein zweites Abbild des Teilgebiets unter einem zweiten Beobachtungswinkel ($\alpha_B$), aus einer zweiten Position ausserhalb des Strahlenbündels (2) erfasst wird, **gekennzeichnet durch**

   - Bestimmung von Augenstrukturen aus dem erfassten ersten Abbild (6A),

   - Bestimmung von Augenstrukturen aus dem erfassten zweiten Abbild (6B),

   - Bestimmung einer ersten Distanz ($d_A$) zwischen den aus dem erfassten ersten Abbild (6A) bestimmten Augenstrukturen,

   - Bestimmung einer zweiten Distanz ($d_B$) zwischen den aus dem erfassten zweiten Abbild (6B) bestimmten Augenstrukturen, und

   - Berechnung von mindestens einem der geometrischen Messwerte (d) aus der bestimmten ersten Distanz ($d_A$) und der bestimmten zweiten Distanz (dB).

14. Verfahren gemäss Anspruch 13, **dadurch gekennzeichnet, dass** die Distanz (d) zwischen den tatsächlichen Augenstrukturen (31, 32) aus der bestimmten ersten Distanz ($d_A$) und der bestimmten zweiten Distanz ($d_B$) berechnet wird.

15. Verfahren gemäss Anspruch 14, **dadurch gekennzeichnet, dass** die Distanz (d) zwischen den tatsächlichen Augenstrukturen (31, 32) durch gewichtete Mittelwertbildung aus der bestimmten ersten Distanz ($d_A$) und der bestimmten zweiten Distanz ($d_B$) berechnet wird.

16. Verfahren gemäss Anspruch 13, **dadurch gekennzeichnet, dass** die Hornhaut des Auges aus dem erfassten ersten Abbild (6A) bestimmt wird, dass die Hornhaut des Auges aus dem erfassten zweiten Abbild (6B) bestimmt wird, dass eine erste Distanz ($d_A$) der aus dem ersten Abbild (6A) bestimmten Hornhaut bestimmt wird, dass eine zweite Distanz ($d_B$) der aus dem zweiten Abbild (6B) bestimmten Hornhaut bestimmt wird, und dass die Dicke (d) der tatsächlichen Hornhaut (3) des Auges aus der bestimmten ersten Distanz ($d_A$) und der bestimmten zweiten Distanz ($d_B$) berechnet wird.

17. Verfahren gemäss Anspruch 13, **dadurch gekennzeichnet, dass** die Hornhaut des Auges aus dem erfassten ersten Abbild (6A) bestimmt wird, dass die Hornhaut des Auges aus dem erfassten zweiten Abbild (6B) bestimmt wird, dass eine erste Distanz ($d_A$) der aus dem ersten Abbild (6A) bestimmten Hornhaut bestimmt wird, dass eine zweite Distanz ($d_B$) der aus dem zweiten Abbild (6B) bestimmten Hornhaut bestimmt wird, und dass ein Neigungswinkels zwischen dem Strahlenbündel (2) und einer Normalen zu der dem Lichtprojektor (11) zugewandten Oberfläche (31) der tatsächlichen Hornhaut (3) des Auges aus der bestimmten ersten Distanz ($d_A$) und der bestimmten zweiten Distanz ($d_B$) berechnet wird.

18. Verfahren gemäss Anspruch 13, **dadurch gekennzeichnet, dass** die erste Position und die zweite Position auf verschiedenen Seiten einer durch das Strahlenbündel (2) gelegten Ebene festgelegt werden und dass der erste ($\alpha_A$) und der zweite Beobachtungswinkel ($\alpha_B$) gleich gross festgelegt werden.

19. Verfahren gemäss Anspruch 13, **dadurch gekennzeichnet, dass** das Strahlenbündel (2) im Wesentlichen senkrecht zu der dem Lichtprojektor (11) zugewandten Oberfläche (31) des Auges projiziert wird.

20. Verfahren gemäss Anspruch 13, **dadurch gekennzeichnet, dass** die Hornhaut des Auges aus dem erfassten ersten Abbild (6A) bestimmt wird, dass die Hornhaut des Auges aus dem erfassten zweiten Abbild (6B) bestimmt wird, dass eine erste Distanz ($d_A$) der aus dem ersten Abbild (6A) bestimmten Hornhaut bestimmt wird, dass eine zweite Distanz ($d_B$) der aus dem zweiten Abbild (6B) bestimmten Hornhaut bestimmt wird, und dass ein Verkippungsfaktor aus der bestimmten ersten Distanz ($d_A$) und der bestimmten zweiten Distanz ($d_B$) berechnet wird, welcher Verkippungsfaktor das Ausmass der Verkippung des Strahlenbündels (2) bezüglich der Normalen zu der dem Lichtprojektor (11) zugewandten Oberfläche (31) der tatsächlichen Hornhaut (3) des Auges und das Ausmass der Abweichung des ersten Beobachtungswinkels ($\alpha_A$) vom zweiten Beobachtungswinkel ($\alpha_B$) angibt.

21. Verfahren gemäss Anspruch 13, **dadurch gekennzeichnet, dass** die Hornhaut des Auges aus dem erfassten ersten Abbild (6A) bestimmt wird, dass die Hornhaut des Auges aus dem erfassten zweiten Abbild (6B) bestimmt wird, dass eine erste Distanz ($d_A$) der aus dem ersten Abbild (6A) bestimmten Hornhaut bestimmt wird, dass eine zweite Distanz ($d_B$) der aus dem zweiten Abbild (6B) bestimmten Hornhaut bestimmt wird, dass die Dicke (d) der tatsächlichen Hornhaut (3) des Auges durch gewichtete Mittelwertbildung aus der bestimmten ersten Distanz ($d_A$) und der bestimmten zweiten Distanz ($d_B$) berechnet wird, dass ein Verkippungsfaktor aus der bestimmten ersten Distanz ($d_A$) und der bestimmten zweiten Distanz ($d_B$) berechnet wird, welcher Verkippungsfaktor das Ausmass der Verkippung des Strahlenbündels (2) bezüglich der Normalen zu der dem Lichtprojektor (11) zugewandten Oberfläche (31) der tatsächlichen Hornhaut (3) des Auges und das Ausmass der Abweichung des ersten Beobachtungswinkels ($\alpha_A$) vom zweiten Beobachtungswinkel ($\alpha_B$) angibt, und dass Gewichtungsfaktoren für die Mittelwertbildung in Abhängigkeit des bestimmten Verkippungsfaktors bestimmt werden.

22. Verfahren gemäss Anspruch 13, **dadurch gekennzeichnet, dass** erste optische Elemente (121A) so bei der ersten Position angeordnet werden, dass sie zur Erfassung des ersten Abbilds (6A) Lichtstrahlen zu einem Bildwandler (120) umlenken, und dass zweite optische Elemente (121B) so bei der zweiten Position angeordnet werden, dass sie zur Erfassung des zweiten Abbilds (6B) Lichtstrahlen zum Bildwandler (120) umlenken.

23. Verfahren gemäss Anspruch 13, **dadurch gekennzeichnet, dass** das Strahlenbündel (2) durch den Lichtprojektor (11) in Form einer Lichtebene projiziert wird.

24. Verfahren gemäss Anspruch 13, **dadurch gekennzeichnet, dass** die Abbilder mittels Bilderfassungsmitteln (120, 121A, 121B, 122A, 122B) in Scheimpfluganordnung zum Strahlenbündel (2) erfasst werden.

25. Verfahren gemäss Anspruch 13, **dadurch gekennzeichnet, dass** Bilderfassungsmittel (120, 121A, 121B, 122A, 122B) zur Erfassung der Abbilder und der Lichtprojektor (11) um eine durch das Strahlenbündel (2) verlaufende Achse (Z) rotiert werden.

**Claims**

1. Device (1) for determining geometric measurement values (d) of an eye, in particular a human eye, comprising:

   - a light projector (11) for projection of a beam of rays (2) through a section of the eye, and

   - imaging means (120, 121A, 121B, 122A, 122B) for capturing a first image (6A) of at least a partial region of the section illuminated by the light projector (11), at a first observation angle ($\alpha_A$), from a first position outside the beam of rays (2), and for capturing a second image (6B) of the partial region at a second observation angle ($\alpha_B$), from a second position outside the beam of rays (2),

**characterised by**:

- image processing means (13) to identify eye structures from the first captured image (6A), to identify eye structures from the second captured image (6B), to measure a first distance ($d_A$) between the eye structures identified from the first image (6A) and to measure a second distance ($d_B$) between the eye structures identified from the second image (6B), and

- processing means (14) to calculate at least one of the geometric measurement values (d) from the measured first distance ($d_A$) and the measured second distance ($d_B$).

2.  Device (1) according to claim 1, **characterised in that** the processing means (14) are designed to calculate a distance (d) between the actual eye structures (31, 32) from the measured first distance ($d_A$) and the measured second distance ($d_B$).

3.  Device (1) according to claim 2, **characterised in that** the processing means (14) are designed to calculate from the measured first distance ($d_A$) and the measured second distance ($d_B$) the distance (d) between the actual eye structures (31, 32) through formation of weighted mean values.

4.  Device (1) according to claim 1, **characterised in that** image processing means (13) are designed to identify the cornea from the first captured image (6A), to identify the cornea from the second captured image (6B), to take a first distance measurement ($d_A$) of the cornea identified from the first image (6A) and to take a second distance measurement ($d_B$) of the cornea identified from the second image (6B), and **in that** the processing means (14) are designed to calculate the thickness (d) of the actual cornea (3) from the first distance measurement ($d_A$) taken and the second distance measurement ($d_B$) taken.

5.  Device (1) according to claim 1, **characterised in that** the image processing means (13) are designed to identify the cornea from the first captured image (6A), to identify the cornea from the second captured image (6B), to take a first distance measurement ($d_A$) of the cornea identified from the first image (6A) and to take a second distance measurement ($d_B$) of the cornea identified from the second image (6B), and **in that** the processing means (14) are designed to calculate from the first distance measurement ($d_A$) taken and the second distance measurement ($d_B$) taken an angle of inclination between the beam of rays (2) and a normal to the surface, turned toward the light projector (11), of the actual cornea.

6.  Device (1) according to claim 1, **characterised in that** the first position and the second position lie on different sides of a plane in which the beam of rays (2) is situated and **in that** the first ($\alpha_A$) and the second observation angles ($\alpha_B$) are equal in size.

7.  Device (1) according to claim 1, **characterised in that** the image processing means (13) are designed to identify the cornea from the first captured image (6A), to identify the cornea from the second captured image (6B), to take a first distance measurement ($d_A$) of the cornea identified from the first image (6A) and to take a second distance measurement ($d_B$) of the cornea identified from the second image (6B), and **in that** the processing means (14) are designed to calculate an angular misalignment factor from the first distance measurement ($d_A$) taken and from the second distance measurement ($d_B$) taken, which angular misalignment factor indicates the magnitude of the angular misalignment of the beam of rays (2) with respect to the normal to the surface (31), turned toward the light projector (11), of the actual cornea (3) and the magnitude of the difference of the first observation angle ($\alpha_A$) from the second observation angle ($\alpha_B$).

8.  Device (1) according to claim 1, **characterised in that** the image processing means (13) are designed to identify the cornea from the first captured image (6A), to identify the cornea from the second captured image (6B), to take a first distance measurement ($d_A$) of the cornea identified from the first image (6A) and to take a second distance measurement ($d_B$) of the cornea identified from the second image (6B), **in that** the processing means (14) are designed to calculate the thickness (d) of the actual cornea (3) from the first distance measurement ($d_A$) taken and the second distance measurement ($d_B$) taken through formation of weighted mean values, **in that** the processing means (14) are designed to calculate an angular misalignment factor from the first distance measurement ($d_A$) taken and the second distance measurement ($d_B$) taken, which angular misalignment factor indicates the magnitude of the angular misalignment of the beam of rays (2) with respect to the normal to the surface (31), turned toward the light projector (11), of the actual cornea (3) and the magnitude of the difference of the first observation angle ($\alpha_A$) from the second observation angle ($\alpha_B$), and **in that** the processing means (14) are designed to determine weighting factors for the formation of mean values depending upon the determined angular misalignment factor.

9.  Device (1) according to claim 1, **characterised in that** the imaging means comprise an image converter (120) and the imaging means comprise optical elements for light ray redirection (121A, 121B), first of said optical elements (121A) being disposed at the first position in such a way that light rays for generation of the first image (6A) are redirected to the image converter (120) and second of said optical elements (121B) being disposed at the second position in such a way that light rays for generation of the second image (6B) are redirected to the image converter (120).

10.  Device (1) according to claim 1, **characterised in that** the light projector (11) is designed such that it projects the beam of rays (2) in the form of a plane of light.

11.  Device (1) according to claim 1, **characterised in that** the imaging means (120, 121A, 121B, 122A, 122B) are disposed in a Scheimpflug configuration with respect to the beam of rays (2).

12.  Device (1) according to claim 1, **characterised in that** it comprises a rotation driver (15) for rotation of the light projector (11) and the imaging means (120, 121A, 121B, 122A, 122B) about an axis (Z) running through the beam of rays (2).

13.  Method for determining geometrical measurement values (d) of an eye, in particular a human eye, in which method a beam of rays (2) is projected through a section of the eye by means of a light projector (11), in which method a first image of at least a partial region of the section illuminated by the light projector (11) is captured at a first observation angle ($\alpha_A$), from a first position outside the beam of rays (2), in which method a second image of the partial region is captured at a second observation angle ($\alpha_B$) from a second position outside the beam of rays (2), **characterised by**:

     -   identifying eye structures from the first captured image (6A),

     -   identifying eye structures from the second captured image (6B),

     -   measuring a first distance ($d_A$) between the eye structures identified from the first captured image (6A),

     -   measuring a second distance ($d_B$) between the eye structures identified from the second captured image (6B), and

     -   calculating from the measured first distance ($d_A$) and the measured second distance ($d_B$) at least one of the geometric measurement values (d).

14.  Method according to claim 13, **characterised in that** the distance (d) between the actual eye structures (31, 32) is calculated from the first measured distance ($d_A$) and the second measured distance ($d_B$).

15.  Method according to claim 14, **characterised in that** the distance (d) between the actual eye structures (31, 32) is calculated from the measured first distance ($d_A$) and the measured second distance ($d_B$) through formation of weighted mean values.

16.  Method according to claim 13, **characterised in that** the cornea is identified from the first captured image (6A), the cornea is identified from the second captured image (6B), a first distance measurement ($d_A$) is taken of the cornea identified from the first image (6A), a second distance measurement ($d_B$) is taken of the cornea identified from the second image (6B), and the thickness (d) of the actual cornea (3) is calculated from the first distance measurement ($d_A$) taken and from the second distance measurement ($d_B$) taken.

17.  Method according to claim 13, **characterised in that** the cornea is identified from the first captured image (6A), the cornea is identified from the second captured image (6B), a first distance measurement ($d_A$) is taken of the cornea identified from the first image (6A), a second distance measurement ($d_B$) is taken of the cornea identified from the second image (6B), and an angle of inclination between the beam of rays (2) and a normal to the surface (31), turned toward the light projector (11), of the actual cornea (3) is calculated from the first distance measurement ($d_A$) taken and the second distance measurement ($d_B$) taken.

18. Method according to claim 13, **characterised in that** the first position and the second position are selected on different sides of a plane in which the beam of rays (2) is situated, and the first ($\alpha_A$) and the second observation angles ($\alpha_B$) are selected as equal in size.

19. Method according to claim 13, **characterised in that** the beam of rays (2) is projected substantially perpendicular to the eye surface turned toward the light projector (11).

20. Method according to claim 13, **characterised in that** the cornea is identified from the first captured image (6A), the cornea is identified from the second captured image (6B), a first distance measurement ($d_A$) is taken of the cornea identified from the first image (6A), a second distance measurement ($d_B$) is taken of the cornea identified from the second image (6B), and an angular misalignment factor is calculated from the first distance measurement ($d_A$) taken and from the second distance measurement ($d_B$) taken, which angular misalignment factor indicates the magnitude of the angular misalignment of the beam of rays (2) with respect to the normal to the surface (31), turned toward the light projector (11), of the actual cornea (3) and the magnitude of the difference of the first observation angle ($\alpha_A$) from the second observation angle ($\alpha_B$).

21. Method according to claim 13, **characterised in that** the cornea is identified from the first captured image (6A), the cornea is identified from the second captured image (6B), a first distance measurement ($d_A$) is taken of the cornea identified from the first image (6A), a second distance measurement ($d_B$) is taken of the cornea identified from the second image (6B), and the thickness (d) of the actual cornea (3) is calculated from the first distance measurement ($d_A$) taken and the second distance measurement ($d_B$) taken through formation of weighted mean values, an angular misalignment factor is calculated from the first distance measurement ($d_A$) taken and from the second distance measurement ($d_B$) taken, which angular misalignment factor indicates the magnitude of the angular misalignment of the beam of rays (2) with respect to the normal to the surface (31), turned toward the light projector (11), of the actual cornea (3) and the magnitude of the difference of the first observation angle ($\alpha_A$) from the second observation angle ($\alpha_B$), and weighting factors for the formation of mean values are determined depending upon the determined angular misalignment factor.

22. Method according to claim 13, **characterised in that** first optical elements (121A) are disposed at the first position in such a way that they redirect light rays to an image converter (120) to capture the first image (6A), and second optical elements (121B) are disposed at the second position in such a way that they redirect light rays to the image converter (120) to capture the second image (6B).

23. Method according to claim 13, **characterised in that** the beam of rays (2) is projected by the light projector (11) in the form of a plane of light.

24. Method according to claim 13, **characterised in that** the images are captured by means of image capturing means (120, 121A, 121B, 122A, 122B) in Scheimpflug configuration with respect to the beam of rays (2).

25. Method according to claim 13, **characterised in that** the image capturing means (120, 121A, 121B, 122A, 122B) for capturing the images and the light projector (11) are rotated about an axis (Z) running through the beam of rays (2).

**Revendications**

1. Dispositif (1) pour la détermination de valeurs géométriques de mesure (d) d'un oeil, en particulier d'un oeil humain, comprenant :

   - un projecteur de lumière (11) pour la projection d'un faisceau de rayon (2) à travers une partie de section transversale de l'oeil, et

   - des moyens de saisie d'images (120, 121A, 121B, 122A, 122B) pour la saisie d'une première image (6) d'au moins une zone partielle de la partie de section transversale illuminée par le projecteur de lumière (11) sous un premier angle d'observation ($\alpha$A), à partir d'une première position à l'extérieur du faisceau de rayon (2) et pour la saisie d'une seconde image (6B) de la zone partielle sous un second angle d'observation ($\alpha$B) à partir d'une seconde position à l'extérieur du faisceau de rayon (2),

**Caractérisé par**

- des moyens de traitement d'image (13) pour la détermination de structures oculaires à partir de la première image saisie (6A), pour la détermination de structures oculaires à partir de la seconde image saisie (6B), pour la détermination d'une première distance (dA) entre les structures oculaires définies à partir de la première image (6A) et pour la détermination d'une seconde distance (dB) entre les structures oculaires définies à partir de la seconde image (6B) et

- des moyens de traitement (14) pour le calcul d'au moins une des valeurs géométriques de mesure (d) à partir de la première distance définie (dA) et de la seconde distance (dB).

2. Dispositif (1) selon la revendication 1, **caractérisé en ce que** les moyens de traitement (14) sont configurés pour le calcul d'une distance (d) entre les structures oculaires effectives (31, 32) à partir de la première distance définie (dA) et la seconde distance définie (dB).

3. Dispositif (1) selon la revendication 2, **caractérisé en ce que** les moyens de traitement (14) sont configurés pour le calcul d'une distance (d) entre les structures oculaires effectives (31, 32) par formation de valeur moyenne pondérée à partir de la première distance définie (dA) et de la seconde distance définie (dB).

4. Dispositif (1) selon la revendication 1, **caractérisé en ce que** les moyens de traitement d'image (13) sont configurés pour la détermination de la cornée de l'oeil à partir de la première image saisie (6A), pour la détermination de la cornée de l'oeil à partir de la seconde image saisie (6B) , pour la détermination d'une première distance (dA) de la cornée définie à partir de la première image (6A) et pour la détermination d'une seconde distance (dB) de la cornée définie à partir de la seconde image (6B) et **en ce que** les moyens de traitement (14) sont configurés pour le calcul de l'épaisseur (d) de la cornée effective (3) de l'oeil à partir de la première distance définie (dA) et de la seconde distance définie (dB).

5. Dispositif (1) selon la revendication 1, **caractérisé en ce que** les moyens de traitement d'image (13) sont configurés pour la détermination de la cornée de l'oeil à partir de la première image saisie (6A), pour la détermination de la cornée de l'oeil à partir de la seconde image saisie (6B) , pour la détermination d'une première distance (dA) de la cornée définie à partir de la première image (6A) et pour la détermination d'une seconde distance (dB) de la cornée définie à partir de la seconde image (6B) et **en ce que** les moyens de traitement (14) sont configurés pour le calcul d'un angle d'inclinaison entre le faisceau de rayon (2) et une normale par rapport à la surface tournée vers le projecteur lumineux (11) de la cornée effective de l'oeil à partir de la première distance définie (dA) et de la seconde distance définie (dB).

6. Dispositif (1) selon la revendication 1, **caractérisé en ce que** la première position et la seconde position se trouvent sur différents côtés d'un plan placé par le faisceau de rayon (2) et **en ce que** le premier ($\alpha$A) et le second angle d'observation ($\alpha$B) sont de même dimension.

7. Dispositif (1) selon la revendication 1, **caractérisé en ce que** les moyens de traitement d'image (13) sont configurés pour la détermination de la cornée de l'oeil à partir de la première image saisie (6A), pour la détermination de la cornée de l'oeil à partir de la seconde image saisie (6B) , pour la détermination d'une première distance (dA) de la cornée définie à partir de la première image (6A) et pour la détermination d'une seconde distance (dB) de la cornée définie à partir de la seconde image (6B) et **en ce que** les moyens de traitement (14) sont configurés pour le calcul d'un facteur d'inclinaison à partir de la première distance définie (dA) et à partir de la seconde distance définie (dB), facteur d'inclinaison qui indique l'ampleur de l'inclinaison du faisceau de rayon (2) par rapport à la normale vis-à-vis de la surface (31) tournée vers le projecteur lumineux (11) de la cornée effective (3) de l'oeil et l'ampleur de la divergence du premier angle d'observation ($\alpha$a) par rapport au premier angle d'observation ($\alpha$B).

8. Dispositif selon la revendication 1, **caractérisé en ce que** les moyens de traitement d'image (13) sont configurés pour la détermination de la cornée de l'oeil à partir de la première image saisie (6A), pour la détermination de la cornée de l'oeil à partir de la seconde image saisie (6B), pour la détermination d'une première distance (dA) de la comée définie à partir de la première image (6A) et pour la détermination d'une seconde distance (dB) de la comée définie à partir de la seconde image (6B) et **en ce que** les moyens de traitement (14) sont configurés pour le calcul de l'épaisseur (d) de la cornée (3) effective de l'oeil par formation d'une valeur moyenne pondérée à partir de la première distance définie (dA) et de la seconde distance (dB), **en ce que** les moyens de traitement (14) sont configurés pour le calcul d'un facteur d'inclinaison à partir de la première distance définie (dA) et à partir de la seconde distance définie (dB), facteur d'inclinaison qui indique l'ampleur de l'inclinaison du faisceau de rayon (2) par rapport à la normale vis-à-vis de la surface (31) tournée vers le projecteur lumineux (11) de la cornée effective (3) de l'oeil et l'ampleur de la divergence du premier angle d'observation ($\alpha$a) par rapport au premier angle d'observation ($\alpha$B) et **en ce que** les moyens de traitement (14) sont configurés pour la détermination de facteurs de pondération pour la formation d'une valeur moyenne en fonction du facteur défini d'inclinaison.

9. Dispositif selon la revendication 1, **caractérisé en ce que** les moyens de saisie d'image comprennent un convertisseur d'image (120) et **en ce que** les moyens de saisie d'image comprennent des éléments optiques pour la déviation du rayon lumineux (121A, 121B), les premiers des moyens optiques (121A) étant disposés dans la première position de sorte que des rayons lumineux sont déviés pour la génération de la première image (6A) en direction du convertisseur d'image (120) et les seconds moyens optiques (121B) étant disposés dans la seconde position de sorte que des rayons lumineux pour des rayons lumineux sont déviés pour la génération de la seconde image (6B) en direction du convertisseur d'image (120).

10. Dispositif selon la revendication 1, **caractérisé en ce que** le projecteur de lumière (11) est conçu de manière à projeter le faisceau de rayon (2) sous forme d'un plan de lumière.

11. Dispositif (1) selon la revendication 1, **caractérisé en ce que** les moyens de saisie d'image (120, 121A, 121B, 122A, 122B) sont disposés dans la structure de Scheimpflug par rapport au faisceau de rayon (2).

12. Dispositif (1) selon la revendication 1, **caractérisé en ce qu'**il comprend un entraînement de rotation (15) pour la rotation du projecteur de lumière (11) et des moyens de saisie d'image (120, 121A, 121b, 122A, 122B) autour d'un axe (2) s'étendant à travers le faisceau de rayon (2).

13. Procédé pour la détermination de valeurs géométriques (d) d'un oeil, en particulier d'un oeil humain, procédé dans lequel un projecteur de lumière (11) projette un faisceau de rayon (2) par une partie de section transversale de l'oeil, procédé dans lequel une première image d'au moins une zone partielle de la partie de section transversale illuminée par le projecteur de lumière (11) sous un premier angle d'observation ($\alpha$A), à partir d'une première position à l'extérieur de faisceau de rayon (2) et pour la saisie d'une seconde image de la zone partielle sous un second angle d'observation ($\alpha$B) à partir d'une seconde position à l'extérieur du faisceau de rayon (2), **caractérisé par**

- la détermination de structures oculaires à partir d'une première image saisie (6A),

- la détermination de structures oculaires à partir d'une seconde image saisie (6B),

- la détermination d'une première distance (dA) entre les structures oculaires définies à partir de la première image saisie (6A),

- la détermination d'une seconde distance (dB) entre les structures oculaires définies à partir de la seconde image saisie (6B), et

- le calcul d'au moins l'une des valeurs géométriques de mesure (d) à partir de la première distance définie (dA) et de la seconde distance définie (dB).

14. 15. Procédé selon la revendication 13, **caractérisé en ce que** la distance (d) entre les structures oculaires effectives (31, 32) sont calculées à partir de la première distance définie (dA) et de seconde distance définie (dB).

15. Procédé selon la revendication 14, **caractérisé en ce que** la distance (d) entre les structures oculaires effectives (31, 32) sont calculées par la formation d'une valeur moyenne pondérée à partir de la première distance définie (dA) et de la seconde distance définie (dB).

16. Procédé selon la revendication 13, **caractérisé en ce que** la cornée de l'oeil est déterminée à partir de la première image saisie (6A), **en ce que** la cornée de l'oeil est déterminée à partir de la seconde distance définie (dB), **en ce qu'**une première distance (dA) de la cornée définie à partir de la première image (6A) est définie, **en ce qu'**une seconde distance (dB) de la cornée définie à partir de la seconde image (6B) est définie, et **en ce que** l'épaisseur (d) de la cornée effective (3) de l'oeil est calculée à partir de la première distance définie (dA) et de la seconde distance définie (dB).

**17.** Procédé selon la revendication 13, **caractérisé en ce que** la cornée de l'oeil est déterminée à partir de la première image saisie (6A), **en ce que** la cornée de l'oeil est déterminée à partir de la seconde distance définie (dB), **en ce qu'**une première distance (dA) de la cornée définie à partir de la première image (6A) est définie, **en ce qu'**une seconde distance (dB) de la cornée définie à partir de la seconde image (6B) est définie, et **en ce qu'**un angle d'inclinaison entre le faisceau de rayon (2) et une normale par rapport à la surface (31) tournée du projecteur lumineux (11), de la comée effective (3) de l'oeil est calculée à partir de la première distance définie (dA) et de la seconde distance définie (dB).

**18.** Procédé selon la revendication 13, **caractérisé en ce que** la première position et la seconde position se trouvent sur différents côtés d'un plan traversant par le faisceau de rayon (2) et **en ce que** le premier ($\alpha$A) et le second angle d'observation ($\alpha$B) sont de même dimension.

**19.** Procédé selon la revendication 13, **caractérisé en ce que** le faisceau de rayon (2) est sensiblement projeté de manière verticale par rapport à la surface (31) de l'oeil, tournée vers le projecteur de lumière (11).

**20.** Procédé selon la revendication 13, **caractérisé en ce que** la cornée de l'oeil est déterminée à partir de la première image saisie (6A), **en ce que** la cornée de l'oeil est déterminée à partir de la seconde image saisie (6B)), **en ce qu'**une première distance (dA) de la cornée définie à partir de la première image (6A) est définie, **en ce qu'**une seconde distance (dB) de la cornée définie à partir de la seconde image (6B) est définie, et **en ce qu'**un facteur d'inclinaison est calculé à partir de la première distance définie (dA) et de la seconde distance définie (dB), facteur d'inclinaison qui indique l'ampleur de l'inclinaison du faisceau de rayon (2) par rapport à la normale vis-à-vis de la surface (31) tournée vers le projecteur lumineux (11) de la cornée effective (3) de l'oeil et l'ampleur de la divergence du premier angle d'observation ($\alpha$a) par rapport au premier angle d'observation ($\alpha$B).

**21.** Procédé selon la revendication 13, **caractérisé en ce que** la cornée de l'oeil est déterminée à partir de la première image saisie (6A), **en ce que** la cornée de l'oeil est déterminée à partir de la seconde image saisie (68)), **en ce qu'**une première distance (dA) de la cornée définie à partir de la première image (6A) est définie, **en ce qu'**une seconde distance (dB) de la cornée définie à partir de la seconde image (6B) est définie, et **en ce que** l'épaisseur (d) de la comée effective (3) de l'oeil est calculée à partir de la première distance définie (dA) et de la seconde distance définie (dB), **en ce qu'**un facteur d'inclinaison est calculé à partir de la première distance définie (dA) et de la seconde distance définie (dB), facteur d'inclinaison qui indique l'ampleur du bascule du faisceau de rayon (2) par rapport à la normale vis-à-vis de la surface (31) tournée vers le projecteur lumineux (11) de la cornée effective (3) de l'oeil et l'ampleur de la divergence du premier angle d'observation ($\alpha$a) par rapport au second angle d'observation ($\alpha$B) et **en ce que** des facteurs de pondération sont définies pour la formation d'une valeur moyenne en fonction du facteur d'inclinaison déterminé.

**22.** Procédé selon la revendication 13, **caractérisé en ce que** des premiers des moyens optiques (121A) sont disposés dans la première position de sorte que des rayons lumineux sont déviés pour la saisie de la première image (6A) en direction d'un convertisseur d'image (120) et **en ce que** les seconds moyens optiques (121B) sont disposés dans la seconde position de sorte que des rayons lumineux sont déviés pour la saisie de la seconde image (6B) en direction du convertisseur d'image (120).

**23.** Procédé selon la revendication 13, **caractérisé en ce que** le faisceau de rayon (2) est projeté par le projecteur de lumière (11) sous forme d'un plan lumineux.

**24.** Procédé selon la revendication 13, **caractérisé en ce que** les images sont saisies à l'aide de moyens de saisie d'image (120, 121A, 121B, 122A, 122B) en disposition de Scheimpflug par rapport au faisceau de rayons (2).

**25.** Procédé selon la revendication 13, **caractérisé en ce que** des moyens de saisie d'image (120, 121A, 121B, 122A, 122B) pour la saisie d'image et le projecteur de lumière (11) sont amenés en rotation autour d'un axe (Z) traversant le faisceau de rayons (2).

FIG. 1

FIG. 2

Z

13  14  15  16

1

10

120

11

121A  121B

122A  122B

$\alpha_A$  $\alpha_B$

2

S  d

3  31

32

**FIG. 3**

62A  62B

61A  $d'_A$  61B

$d_A$  $\vartheta$  $d_B$

6A  6B

**FIG. 4**